# EUROPEAN PATENT SPECIFICATION

(11) **EP 2 338 959 B1**
(45) Date of publication and mention of the grant of the patent: **12.09.2018**
(21) Application number: 10014927.7
(22) Date of filing: 26.06.2006
(51) Int. Cl.: C10M 171/00, C09K 5/04

(54) **COMPOSITIONS CONTAINING FLUORINE SUBSTITUTED OLEFINS**
ZUSAMMENSETZUNGEN MIT FLUORSUBSTITUIERTEN OLEFINEN
COMPOSITIONS CONTENANT DES OLÉFINES SUBSTITUÉES DE FLUORINE

(30) Priority: 24.06.2005 US 693853 P
(43) Date of publication of application: 29.06.2011
(62) Divisional of application: 06785612.0
(73) Proprietor: Honeywell International Inc., Morris Plains, NJ 07950 (US)
(72) Inventor: Pham, Hang T, Morristown, NJ 07962-2245 (US); Singh, Rajiv R, morristown, NJ 07962-2245 (US)
(74) Representative: Crooks, Elizabeth Caroline

(56) References cited:
- US-A1- 2004 089 839
- US-A1- 2004 256 594
- DATABASE WPI Week 199221 Thomson Scientific, London, GB; AN 1992-172539 XP2343594, -& JP H04 110388 A (DAIKIN KOGYO KK) 10 April 1992 (1992-04-10)

## Description

### FIELD OF THE INVENTION

This invention relates to compositions, having utility as a compatibiliser.

### BACKGROUND

Fluorocarbon based fluids have found widespread use in many commercial and industrial applications, including as the working fluid in systems such as air conditioning, heat pump and refrigeration systems, as aerosol propellants, as blowing agents, as heat transfer media, and as gaseous dielectrics. Because of certain suspected environmental problems, including the relatively high global warming potentials, associated with the use of some of the compositions that have heretofore been used in these applications, it has become increasingly desirable to use fluids having low or even zero ozone depletion potential, such as hydrofluorocarbons ("HFCs"). Thus, the use of fluids that do not contain chlorofluorocarbons ("CFCs") or hydrochlorofluorocarbons ("HCFCs") is desirable. Furthermore, some HFC fluids may have relatively high global warming potentials associated therewith, and it is desirable to use hydrofluorocarbon or other fluorinated fluids having as low global warming potentials as possible while maintaining the desired performance in use properties. Additionally, the use of single component fluids or azeotrope-like mixtures, which do not substantially fractionate on boiling and evaporation, is desirable in certain circumstances.

Certain fluorocarbons have been a preferred component in many heat exchange fluids, such as refrigerants, for many years in many applications. For, example, fluoroalkanes, such as chlorofluoromethane and chlorofluoroethane derivatives, have gained widespread use as refrigerants in applications including air conditioning and heat pump applications owing to their unique combination of chemical and physical properties. Many of the refrigerants commonly utilized in vapor compression systems are either single components fluids or azeotropic mixtures.

As suggested above, concern has been increasing in recent years about potential damage to the earth's atmosphere and climate, and certain chlorine-based compounds have been identified as particularly problematic in this regard. The use of chlorine-containing compositions (such as chlorofluorocarbons (CFC's), hydrochlorofluorocarbons (HCF's) and the like) as the working fluid in heat transfer systems, such as in refrigeration and air-conditioning systems, has become disfavored because of the ozone-depleting properties associated with many of such compounds. There has thus been an increasing need for new fluorocarbon and hydrofluorocarbon compounds and compositions that are attractive alternatives to the compositions heretofore used in these and other applications. For example, it has become desirable to retrofit chlorine-containing refrigeration systems by replacing chlorine-containing refrigerants with non-chlorine-containing refrigerant compounds that will not deplete the ozone layer, such as hydrofluorocarbons (HFC's). Industry in general and the heat transfer industry in particular are continually seeking new fluorocarbon based mixtures that offer alternatives to, and are considered environmentally safer substitutes for, CFCs and HCFCs. It is generally considered important, however, at least with respect to heat transfer fluids, that any potential substitute must also possess those properties present in many of the most widely used fluids, such as excellent heat transfer properties, chemical stability, low- or no- toxicity, non-flammability and/or lubricant compatibility, among others.

Applicants have come to appreciate that lubricant compatibility is of particular importance in many of applications. More particularly, it is highly desirably for refrigeration fluids to be compatible with the lubricant utilized in the compressor unit, used in most refrigeration systems. Unfortunately, many non-chlorine-containing refrigeration fluids, including HFC's, are relatively insoluble and/or immiscible in the types of lubricants used traditionally with CFC's and HFC's, including, for example, mineral oils, alkylbenzenes or poly(alpha-olefins). In order for a refrigeration fluid-lubricant combination to work at a desirable level of efficiently within a compression refrigeration, air-conditioning and/or heat pump system, the lubricant should be sufficiently soluble in the refrigeration liquid over a wide range of operating temperatures. Such solubility lowers the viscosity of the lubricant and allows it to flow more easily throughout the system. In the absence of such solubility, lubricants tend to become lodged in the coils of the evaporator of the refrigeration, air-conditioning or heat pump system, as well as other parts of the system, and thus reduce the system efficiency.

With regard to efficiency in use, it is important to note that a loss in refrigerant thermodynamic performance or energy efficiency may have secondary environmental impacts through increased fossil fuel usage arising from an increased demand for electrical energy.

Furthermore, it is generally considered desirably for CFC refrigerant substitutes to be effective without major engineering changes to conventional vapor compression technology currently used with CFC refrigerants.

Flammability is another important property for many applications. That is, it is considered either important or essential in many applications, including particularly in heat transfer applications, to use compositions which are non-flammable. Thus, it is frequently beneficial to use in such compositions compounds which are nonflammable. As used herein, the term "nonflammable" refers to compounds or compositions which are determined to be nonflammable as determined in accordance with ASTM standard E-681, dated 2002, which is incorporated herein by reference. Unfortunately, many HFC's which might otherwise be desirable for used in refrigerant compositions are not nonflammable. For example, the fluoroalkane difluoroethane (HFC-152a) and the fluoroalkene 1,1,1-trifluorpropene (HFO-1243zf) are each flammable and therefore not viable for use in many applications.

Higher fluoroalkenes, that is fluorine-substituted alkenes having at least five carbon atoms, have been suggested for use as refrigerants. U.S. Patent No. 4,788,352 - Smutny is directed to production of fluorinated C₅ to C₈ compounds having at least some degree of unsaturation. The Smutny patent identifies such higher olefins as being known to have utility as refrigerants, pesticides, dielectric fluids, heat transfer fluids, solvents, and intermediates in various chemical reactions. (See column 1, lines 11 - 22).

While the fluorinated olefins described in Smutny may have some level of effectiveness in heat transfer applications, it is believed that such compounds may also have certain disadvantages. For example, some of these compounds may tend to attack substrates, particularly general-purpose plastics such as acrylic resins and ABS resins. Furthermore, the higher olefinic compounds described in Smutny may also be undesirable in certain applications because of the potential level of toxicity of such compounds which may arise as a result of pesticide activity noted in Smutny. Also, such compounds may have a boiling point which is too high to make them useful as a refrigerant in certain applications.

Bromofluoromethane and bromochlorofluoromethane derivatives, particularly bromotrifluoromethane (Halon 1301) and bromochlorodifluoromethane (Halon 1211) have gained widespread use as fire extinguishing agents in enclosed areas such as airplane cabins and computer rooms. However, the use of various halons is being phased out due to their high ozone depletion. Moreover, as halons are frequently used in areas where humans are present, suitable replacements must also be safe to humans at concentrations necessary to suppress or extinguish fire.

US 2004/256594 discloses the use of tetrafluoropropenes in a variety of applications, including as compatibilising agents.

Applicants have thus come to appreciate a need for compositions, and particularly compatabilizing agents, that are potentially useful in numerous applications, while avoiding one or more of the disadvantages noted above.

### SUMMARY

Applicants have found that the above-noted need, and other needs, can be satisfied by compatibiliser compositions, comprising 1,1,1-trifluoro-3-chloropropene (HFCO-1233zd)

The term "HFCO-1233zd" is used herein to refer to both cis- and trans-1,1,1-trifluo-3,chlororopropene (HFCO-1233zd). The term HFCO-1233zd is used herein generically to refer to 1,1,1-trifluo-3,chloropropene, independent of whether it is the cis- or trans- form. The terms "cisHFCO- 1233zd" and "transHFCO-1233zd" are used herein to describe the cis- and trans- forms of 1, 1, 1-trifluo,3-chlororopropene, respectively. The term "HFCO-1233zd" therefore includes within its scope cisHFCO-1233zd, transHFCO-1233zd, and all combinations and mixtures of these.

The present invention provides also methods and systems which utilize the compositions of the present invention as compatibilisers.

### DETAILED DESCRIPTION OF PREFERRED EMBODIMENTS

### THE COMPOSITIONS

Applicants believe that, in general, HFCO-1233zd is generally effective and exhibit utility as compatibilzers. However, applicants have surprisingly and unexpectedly found that HFCO-1233zd exhibits a highly desirable low level of toxicity compared to other of such compounds. As can be readily appreciated, this discovery is of potentially enormous advantage and benefit for the formulation of compositions which would otherwise contain relatively toxic compounds. Applicants believe also that all structural, geometric and stereoisomers of HFCO-1233zd are effective and of beneficially low toxicity.

The present compositions, comprising HFCO-1233zd are believed to possess properties that are advantageous for a number of important reasons. For example, applicants believe, based at least in part on mathematical modeling, that HFCO-1233zd will not have a substantial negative affect on atmospheric chemistry, being negligible contributors to ozone depletion in comparison to some other halogenated species. The preferred compositions of the present invention thus have the advantage of not contributing substantially to ozone depletion. The preferred compositions also do not contribute substantially to global warming compared to many of the hydrofluoroalkanes presently in use.

Of course other compounds and/or components that modulate a particular property of the compositions (such as cost for example) may also be included in the present compositions, and the presence of all such compounds and components is within the broad scope of the invention.

In certain preferred forms, compositions of the present invention have a Global Warming Potential (GWP) of not greater than about 1000, more preferably not greater than about 500, and even more preferably not greater than about 150. In certain embodiments, the GWP of the present compositions is not greater than about 100 and even more preferably not greater than about 75. As used herein, "GWP" is measured relative to that of carbon dioxide and over a 100 year time horizon, as defined in "The Scientific Assessment of Ozone Depletion, 2002, a report of the World Meteorological Association's Global Ozone Research and Monitoring Project,".

In certain preferred forms, the present compositions also preferably have an Ozone Depletion Potential (ODP) of not greater than 0.05, more preferably not greater than 0.02 and even more preferably about zero. As used herein, "ODP" is as defined in "The Scientific Assessment of Ozone Depletion, 2002, A report of the World Meteorological Association's Global Ozone Research and Monitoring Project,".

The amount of HFCO-1233zd, contained in the present compositions can vary widely, depending the particular application, and compositions containing more than trace amounts and less than 100% of the compound are within broad the scope of the present invention. Moreover, the compositions of the present invention can be azeotropic, azeotrope-like or non-azeotropic. In preferred embodiments, the present compositions comprise HFCO-1233zd in amounts from about 5% by weight to about 99% by weight, and even more preferably from about 5% to about 95%. Many additional compounds or components, including lubricants, stabilizers, metal passivators, corrosion inhibitors, flammability suppressants, and other compounds and/or components that modulate a particular property of the compositions (such as cost for example) may be included in the present compositions, and the presence of all such compounds and components is within the broad scope of the invention. In certain preferred embodiments, the present compositions include, in addition to the HFCO-1233zd one or more of the following:
Trichlorofluoromethane (CFC-11)
Dichlorodifluoromethane (CFC-12)
Difluoromethane (HFC-32)
Pentafluoroethane (HFC-125)
1,1,2,2-tetrafluoroethane (HFC-134)
1,1,1,2-Tetrafluoroethane (HFC-134a)
Difluoroethane (HFC-152a)
1,1,1,2,3,3,3-Heptafluoropropane (HFC-227ea)
1,1,1,3,3,3-hexafluoropropane (HFC-236fa)
1,1,1,3,3-pentafluoropropane (HFC-245fa)
1,1,1,3,3-pentafluorobutane (HFC-365mfc)
water
CO₂

The relative amount of HFCO-1233zd as well as any additional components which may be included in present compositions, can vary widely within the general broad scope of the present invention according to the particular application for the composition, and all such relative amounts are considered to be within the scope hereof.

Accordingly, applicants have recognized that certain compositions of the present invention can be used to great advantage in compatibilizer application. It is believed that those of skill in the art will be readily able to adapt the present compositions for use in this application without undue experimentation.

The present compositions are generally useful as replacements for CFCs, such as dichlorodifluormethane (CFC-12), HCFCs, such as chlorodifluoromethane (HCFC-22), HFCs, such as tetrafluoroethane (HFC-134a), and combinations of HFCs and CFCs, such as the combination of CFC-12 and 1,1-difluorethane (HFC-152a) (the combination CFC-12:HFC-152a in a 73.8:26.2 mass ratio being known as R-500) in refrigerant, aerosol, and other applications.

Furthermore, the present compositions may also include a compatibilzer, such as propane, for the purpose of aiding compatibility and/or solubility of the lubricant. Such compatibilizers, including propane, butanes and pentanes, are preferably present in amounts of from about 0.5 to about 5 percent by weight of the composition.

## Claims

1. Use as a compatibiliser of a composition comprising at least one fluoroalkene, wherein the at least one fluoroalkene comprises 1,1,1-trifluoro-3-chloropropene (HFCO-1233zd).

2. Use according to claim 1, wherein the at least one fluoroalkene comprises cis HFCO-1233zd, trans HFCO-1233zd or a combination thereof.

3. Use according to claim 2, wherein the at least one fluoroalkene comprises trans HFCO-1233zd.

4. Use according to any preceding claim, wherein the composition comprises from about 5 % to about 99 %, preferably from about 5 % to about 95 %, by weight of the at least one fluoroalkene.

5. Use according to any preceding claim, wherein the composition has a Global Warming Potential (GWP) of not greater than about 500, and preferably not greater than about 150.

6. Use according to any preceding claim wherein the composition further comprises a compatibiliser selected from propanes, butanes or pentanes.

## Patentansprüche

1. Verwendung einer Zusammensetzung, die mindestens ein Fluoralken umfasst, als Verträglichkeitsvermittler, wobei das mindestens eine Fluoralken 1,1,1-Trifluor-3-chlorpropen (HFCO-1233zd) umfasst.

2. Verwendung nach Anspruch 1, wobei das mindestens eine Fluoralken cis-HFCO-1233zd, trans-HFCO-1233zd oder eine Kombination davon umfasst.

3. Verwendung nach Anspruch 2, wobei das mindestens eine Fluoralken trans-HFCO-1233zd umfasst.

4. Verbindung nach einem der vorhergehenden Ansprüche, wobei die Zusammensetzung etwa 5 bis etwa 99 Gew.-%, vorzugsweise etwa 5 bis etwa 95 Gew.-%, des mindestens einen Fluoralkens umfasst.

5. Verwendung nach einem der vorhergehenden Ansprüche, wobei die Zusammensetzung ein Treibhauspotential (Global Warming Potential, GWP) von nicht mehr als etwa 500 und vorzugsweise nicht mehr als etwa 150 aufweist.

6. Verwendung nach einem der vorhergehenden Ansprüche, wobei die Zusammensetzung ferner einen aus Propanen, Butanen oder Pentanen ausgewählten Verträglichkeitsvermittler umfasst.

## Revendications

1. Utilisation en tant que compatibilisateur d'une composition comprenant au moins un fluoroalcène, dans laquelle l'au moins un fluoroalcène comprend le 1,1,1-trifluoro-3-chloropropène (HFCO-1233zd).

2. Utilisation selon la revendication 1, dans laquelle l'au moins un fluoroalcène comprend cis-HFCO-1233zd, trans-HFCO-1233zd ou une combinaison de ceux-ci.

3. Utilisation selon la revendication 2, dans laquelle l'au moins un fluoroalcène comprend trans-HFCO-1233zd.

4. Utilisation selon l'une quelconque des revendications précédentes, dans laquelle la composition comprend d'environ 5 % à environ 99 %, de préférence d'environ 5 % à environ 95 %, en poids de l'au moins un fluoroalcène.

5. Utilisation selon l'une quelconque des revendications précédentes, dans laquelle la composition présente un potentiel de réchauffement global (PRG) de pas plus d'environ 500, et de préférence pas plus d'environ 150.

6. Utilisation selon l'une quelconque des revendications précédentes dans laquelle la composition comprend en outre un compatibilisateur choisi parmi les propanes, butanes ou pentanes.
